(19) Europäisches Patentamt
European Patent Office
Office européen des brevets

(11) Veröffentlichungsnummer: **0 138 141**
**B1**

(12) # EUROPÄISCHE PATENTSCHRIFT

(45) Veröffentlichungstag der Patentschrift: **24.02.88**

(21) Anmeldenummer: **84111738.5**

(22) Anmeldetag: **02.10.84**

(51) Int. Cl.⁴: **C 07 B 35/02,** C 07 F 15/00 // C07C119/08, C07C121/16, C07C121/48, C07C131/00, C07C120/00

(54) **Verfahren zur selektiven Hydrierung von C-C-Doppelbindungen in Gegenwart von reduzierbaren, stickstoffhaltigen Gruppen.**

(30) Priorität: **13.10.83 DE 3337294**

(43) Veröffentlichungstag der Anmeldung: **24.04.85 Patentblatt 85/17**

(45) Bekanntmachung des Hinweises auf die Patenterteilung: **24.02.88 Patentblatt 88/8**

(84) Benannte Vertragsstaaten: **BE CH DE FR GB IT LI NL**

(56) Entgegenhaltungen:
**US-A-3 454 644**
**US-A-3 488 400**
**US-A-3 489 786**

(73) Patentinhaber: **BAYER AG, Konzernverwaltung RP Patentabteilung, D-5090 Leverkusen 1 Bayerwerk (DE)**

(72) Erfinder: **Fiedler, Paul, Dr., Rybnikerstrasse 8, D-5000 Köln 80 (DE)**
Erfinder: **Braden, Rudolf, Dr., Nothauserfeld 1, D-5068 Odenthal (DE)**
Erfinder: **Buding, Hartmuth, Dr., Liebermannstrasse 1, D-4047 Dormagen 1 (DE)**

**EP 0 138 141 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur selektiven katalytischen Hydrierung von C-C-Doppelbindungen in Gegenwart von reduzierbaren, stickstoffhaltigen Gruppen.

Es ist bekannt, daß man C-C-Doppelbindungen selektiv neben stickstoffhaltigen, reduzierbaren Gruppen an festen Katalysatoren hydrieren kann. Dazu verwendet man Palladium- oder Platin-Katalysatoren. Wenn die olefinischen Doppelbindungen nicht stark substituiert sind, können sie z. B. ohne Angriff auf eine Cyangruppe hydriert werden. Es werden dabei Ausbeuten bis zu 90 % erreicht (s. Houben-Weyl, Methoden der Organischen Chemie, Band IV, 1c, Reduktion I (1980), Seite 168). Die Selektivität ist jedoch häufig unbefriedigend. So erhält man bei Verwendung von Platinoxid zur Hydrierung von 1-Cyanocyclohexen nur 31 % des gewünschten Cyanocyclohexans (s. M. Freifelder, Practical Catalytic Hydrogenation, (1971), Seite 157).

Die Hydrierung von ungesättigten Nitrilen unter Verwendung von Rhodiumkomplexen als homogene Katalysatoren geht auf G. Wilkinson zurück. Die heterofunktionelle Cyangruppe wird bei der Hydrierung mit dem Wilkinson-Komplex als Katalysator nicht hydriert. Infolge Ligandenaustausch kann mit Nitrilen aber eine Entaktivierung des Katalysators erfolgen (s. Houben-Weyl, a.a.0., Seiten 57 bis 60).

Da Rhodiumkomplexe der Formel $[(C_6H_5)_3P]_3Rh^IX$ auch für die Hydrierung von Nitrilen zu Aminen geeignet sind (s. DE-AS 17 93 616, Spalte 2, Zeile 51) muß man erwarten, daß ihre Selektivität für die Hydrierung von olefinischen Doppelbindungen neben Nitrilgruppen nicht immer ausreicht.

Von halogenhaltigen Rutheniumkomplexen ist zwar bekannt, daß sie vornehmlich endständige Olefinbindungen hydrieren, eine selektive Hydrierung, insbesondere von innenständigen Doppelbindungen neben stickstoffhaltigen funktionellen Gruppen ist jedoch bisher nicht beschrieben. Dagegen ist bekannt, daß Rutheniumkomplexe unter Hydrierbedingungen bereits ab 80°C Angriffe auf funktionelle Gruppen zeigen (s. Houben-Weyl, a.a.0., Seite 56).

Aus der US-PS 3 454 644 ist bekannt, daß man mit phosphinhaltigen Rutheniumkomplexen des Typs $L_nMX_2$ (L = CO oder tertiäres Phosphin, n = 3 oder 4, M = Ruthenium oder Osmium, X = Halogen und/oder Wasserstoff) Keto-, Formyl-, Nitril-, nichtaromatische -C=C- und -C≡C-Gruppen hydrieren kann, wobei stets sämtliche vorhandene Gruppen dieser Art hydriert werden.

Von Rutheniumkomplexen ist eine befriedigende Selektivität für die Hydrierung von C-C-Doppelbindungen, insbesondere von nicht-endständigen Doppelbindungen, neben reduzierbaren, stickstoffhaltigen, funktionellen Gruppen deshalb nicht zu erwarten.

Es wurde nun ein Verfahren zur selektiven Hydrierung von Kohlenstoff-Kohlenstoff-Doppelbindungen in Gegenwart reduzierbarer, stickstoffhaltiger Gruppen gefunden, das dadurch gekennzeichnet ist, daß man die Hydrierung in Gegenwart eines katalytisch wirksamen Rutheniumkomplexes durchführt, der der Formel

$RuXL_1(L_2)_2$ (I)

entspricht, in der

X für Chlor, Brom, Jod oder Wasserstoff steht,

$L_1$ für einen aromatischen Liganden der Formel

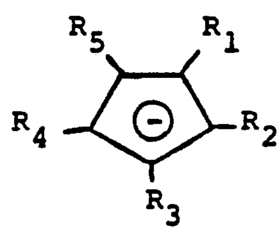

(II)

steht, in der $R_1$ bis $R_5$ gleich oder verschieden sein können und für Wasserstoff, Methyl, Ethyl oder Phenyl stehen, wobei auch je zwei benachbarte Reste aus der Gruppe $R_1$ bis $R_5$ gemeinsam einen Kohlenwasserstoffrest derart bilden können, daß $L_1$ insgesamt ein kondensiertes Ringsystem darstellt, das gegebenenfalls unter Reaktionsbedingungen inerte Substituenten enthält und

$L_2$ für einen Liganden aus der Gruppe der tertiären Organophosphor-, Organoarsen- und Organoantimon-Verbindungen steht oder

$(L_2)_2$ für einen zweizähnigen Bisphosphan-Liganden steht.

In Formel (I) steht X vorzugsweise für Wasserstoff oder Chlor.

Beispiele für $L_1$-Liganden sind Cyclopentadienyl, Pentamethylcyclopentadienyl, Ethyltetramethylcyclopentadienyl, Pentaphenylcyclopentadienyl und Dimethyltriphenylcyclopentadienyl. Bevorzugter $L_1$-Ligand ist Cyclopentadienyl.

Beispiele für $L_2$-Liganden sind solche, die den Formeln

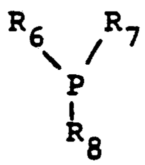 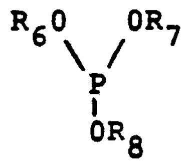 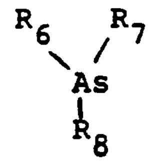 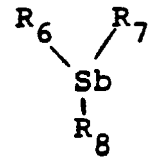

(III) (IV) (V) (VI)

entsprechen, in denen $R_6$, $R_7$ und $R_8$ gleich oder verschieden sein können und Alkyl-, Cycloalkyl-, Aryl- oder Aralkylreste bedeuten, wobei diese Reste gegebenenfalls durch Alkyl-, Hydroxy-, Alkoxy-, Carboalkoxy- oder Halogengruppen substituiert sein können.

Alkylreste sind hier beispielsweise geradkettige oder verzweigte, gesättigte Kohlenwasserstoffreste mit 1 bis 20, bevorzugt 1 bis 12, besonders bevorzugt 1 bis 6 C-Atomen. Besonders bevorzugte Alkylreste sind z. B. Methyl, Ethyl, Propyl, Isopropyl, Butyl, Isobutyl, Pentyl, Isopentyl, Hexyl und Isohexyl.

Cycloalkylreste sind hier beispielsweise cyclische, gesättigte Kohlenwasserstoffreste mit 5 bis 12, bevorzugt 5 bis 7 C-Atomen, wie Cyclopentyl, Cyclohexyl oder Cycloheptyl.

Arylreste sind hier beispielsweise aromatische Kohlenwasserstoffreste aus der Benzolreihe mit 6 bis 18 bevorzugt 6 bis 12 C-Atomen, beispielsweise Phenyl, Biphenyl, Naphthyl und Anthracyl.

Aralkylreste sind hier beispielsweise durch Aryl substituierte Alkylreste, die im aliphatischen Teil aus einem geradkettigen oder verzweigten Kohlenwasserstoffrest mit 1 bis 6 C-Atomen und im aromatischen Teil aus einem Rest der Benzolreihe, vorzugsweise Phenyl, bestehen. Ein Beispiel hierfür ist der Benzylrest.

Die vorstehend erläuterten Alkyl-, Cycloalkyl-, Aryl- und Aralkylreste können gegebenenfalls durch $C_1$- bis $C_6$-Alkyl, Hydroxy, $C_1$- bis $C_6$-Alkoxy, $C_1$- bis $C_6$-Carbalkoxy, Fluor oder Chlor substituiert sein. Beispiele für Alkylsubstituenten sind Methyl, Ethyl, Propyl, Isopropyl, Butyl, Isobutyl, Pentyl, Isopentyl, Hexyl und Isohexyl.

Beispiele für Alkoxysubstituenten sind Methoxy, Ethoxy, Propoxy, Isopropoxy, Butoxy, Isobutoxy, Pentoxy, Isopentoxy, Hexoxy und Isohexoxy. Beispiele für Carbalkoxysubstituenten sind Carbmethoxy, Carbethoxy, Carbisopropoxy und Carbpropoxy. Von den Substituenten Fluor und Chlor ist Fluor bevorzugt.

Bevorzugte $L_2$-Liganden sind solche der Formel (III). Beispiele hierfür sind Triphenylphosphan, Diethylphenylphosphan, Tritolylphosphan, Trinaphthylphosphan, Diphenylmethylphosphan, Diphenylbutylphosphan, Tris-(p-carbomethoxyphenyl)-phosphan, Tris-(p-cyanophenyl)-phosphan, Tributylphosphan, Tris-(trimethylphenyl)-phosphane, Tris-(trimethoxyphenyl)-phosphane, Bis-(trimethylphenyl)-phenylphosphane, Bis-(trimethoxyphenyl)-phenylphosphane, Trimethylphenyldiphenylphosphane, Trimethoxyphenyldiphenylphosphane, Tris-(dimethylphenyl)phenylphosphane, Tris-(dimethoxyphenyl)-phosphane, Bis-(dimethylphenyl)-phenylphosphane, Bis-(dimethoxyphenyl)-phenylphosphane, Dimethylphenyldiphenylphosphane und Dimethoxyphenyldiphenylphosphane.

Wenn die Gruppe $(L_2)_2$ in der Formel (I) für einen zweizähnigen Bisphosphan-Liganden steht, so kann dieser beispielsweise der Formel

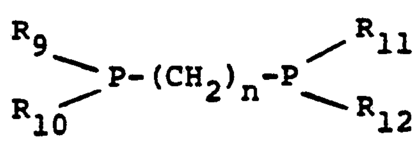 (VIII)

entsprechen, in der n für eine ganze Zahl von 1 bis 10 steht und die Reste $R_9$, $R_{10}$, $R_{11}$ und $R_{12}$ gleich oder verschieden sein können und in ihrer Bedeutung den Resten $R_6$, $R_7$ und $R_8$, wie sie für Formeln (III) bis (VI) beschrieben sind, entsprechen. Beispiele für Bisphosphane dieser Gruppe sind 1,2-Bisdianisylphosphanoethan oder 1,4-Bis-diphenylphosphanobutan.

Rutheniumkomplexe der Formel

$RuXL_1(L_2)_2$ (I),

bei denen

$L_1$ Cyclopentadienyl,

$L_2$ einfache Phosphane oder Phosphite, wie Triphenylphosphan, Tritolylphosphan oder Trimethylphosphit, bedeuten und

X für Chlor, Brom, Jod oder Wasserstoff steht, sind zum Teil bekannt (s. M.I. Bruce, N.J. Windsor, Aust. J. Chem. 30 (1977), Seiten 1601 bis 1604).

Die Rutheniumkomplexe der Formel

$RuXL_1(L_2)_2$ (I)

mit X=Wasserstoff können z. B. durch Hydrierung der entsprechenden Rutheniumkomplexe der Formel (I) mit X=Chlor erhalten werden. Diese Hydrierung kann beispielsweise durch Umsetzung mit Lithiumalanat in Ether erfolgen (s. T. Blackmore, M.I. Bruce und F.G.A. Stoue, J. Chem. Soc. Sect. A 1971, S. 2376 bis 2382), oder durch Umsetzung mit Natriummethylat in Methanol (s. T. Wilczewsky, M. Bochenska und J.F. Biernat, J. Organomet. Chem. 215 (1981), S. 87 bis 96).

Die Rutheniumkomplexe der Formel

$RuXL_1(L_2)_2$ (I)

mit X=Chlor können beispielsweise durch Umsetzung von hydratisiertem Rutheniumtrichlorid mit einem Überschuß der Liganden $L_1$ und $L_2$ in Ethanol erhalten werden (s. M.I. Bruce und N.J. Windsor, Aust. J. Chem. 30 (1977), Seiten 1601 bis 1604).

Die Rutheniumkomplexe der Formel

$RuXL_1(L_2)_2$ (I)

mit X=Brom oder Jod können z. B. durch einfaches Erhitzen der entsprechenden Rutheniumkomplexe der Formel (I) mit X=Wasserstoff mit HBr oder HJ in Methanol gewonnen werden (s. T. Wilczewsky, M. Bochenska, J.F. Biernat in J. Organomet. Chem. 215 (1981), S. 87 bis 96).

Für die Durchführung des erfindungsgemäßen Verfahrens kann man beispielsweise 0,001 bis 50 mMol eines katalytisch wirksamen Rutheniumkomplexes der vorstehend beschriebenen Art pro Mol zu hydrierender C-C-Doppelbindung einsetzen. Bevorzugt wendet man Katalysatormengen von 0,01 bis 10 Mmol pro Mol zu hydrierender C-C-Doppelbindung an. Das erfindungsgemäße Verfahren wird im allgemeinen im Temperaturbereich von 20 bis 250°C durchgeführt. Bevorzugt sind Temperaturen im Bereich von 80 bis 150°C.

Das erfindungsgemäße Verfahren wird im allgemeinen im Druckbereich von 5 bis 300 bar durchgeführt. Es sind auch Drucke unterhalb 5 bar möglich, wobei dann allerdings meistens die Reaktionszeit verlängert werden muß. Ebenso kann das erfindungsgemäße Verfahren auch bei höheren Drucken als 300 bar durchgeführt werden, was aber mit einem erhöhten technischen Aufwand verbunden ist. Bevorzugt wird das erfindungsgemäße Verfahren im Druckbereich zwischen 20 und 200 bar durchgeführt.

Vorzugsweise werden in das erfindungsgemäße Verfahren Verbindungen der Formeln (III), (IV), (V) und (VI) nur in Form der Rutheniumkomplexe der Formeln (I) bzw. (VII) eingebracht, das bedeutet, nicht in freier Form.

Das erfindungsgemäße Verfahren wird im allgemeinen in flüssiger Phase durchgeführt. Das flüssige Reaktionsmedium kann ein Gemisch von an sich vorhandenen Flüssigkeiten sein, d. h. ein Gemisch von Einsatz- und Reaktionsprodukten, oder, insbesondere, wenn die Einsatz- und/oder Reaktionsprodukte unter Reaktionsbedingungen fest sind, aus einem zugesetzten Lösungsmittel bestehen, das unter den Reaktionsbedingungen inert ist.

Wenn das erfindungsgemäße Verfahren in Gegenwart eines Lösungsmittels durchgeführt werden soll, kann man beispielsweise organische Lösungsmittel zusetzen, die sich unter den Reaktionsbedingungen nicht verändern. Beispielsweise kommen hierfür in Betracht Halogen-, Alkyl- oder Alkoxy-substituierte Benzole, wie Chlorbenzol, Toluol, Xylole und Anisol; Ester, wie Essigester oder Adipinsäuredimethylester; Ester und Ether von Polyolen, wie Glykoldiacetat oder Tetraglyme; cyclische Ether, wie Tetrahydrofuran und Dioxan; niedere Alkohole, wie Methanol und Isopropanol; oder Ketone, wie Aceton, Butanon, Diisopropylketon und Cyclohexanon. Bevorzugte Lösungsmittel sind Toluol, Isopropanol und Butanon.

Die Lösungsmittel können beispielsweise im Gewichtsverhältnis von 30 : 1 bis 1 : 10, bezogen auf das Ausgangsprodukt, angewendet werden. Bevorzugt ist dieses Verhältnis 15 : 1 bis 1 : 3.

Mit dem erfindungsgemäßen Verfahren können die verschiedensten Verbindungen, die C-C-Doppelbindungen und reduzierbare, stickstoffhaltige Gruppen enthalten, selektiv an den C-C-Doppelbindungen hydriert werden. Als reduzierbare, stickstoffhaltige Gruppen können beispielsweise Nitril-, aber auch Oximin- und/oder Imingruppen vorhanden sein. Es können mehrere, auch verschiedenartige, reduzierbare, stickstoffhaltige Gruppen vorhanden sein. Bei den C-C-Doppelbindungen kann es sich um olefinische Doppelbindungen handeln, die in offenkettigen Verbindungen innen- oder endständig oder in cyclischen Verbindungen vorliegen können. Beispiele für in erfindungsgemäßer Weise selektiv hydrierbare Verbindungen sind Alkennitrile, Alkendinitrile, Alkencarbaldoxime. Alkencarbaldimine, die jeweils vorzugsweise 2 bis 20 C-Atome enthalten, sowie Cycloalkennitrile, Cycloalkencarbaldoxime, Cycloalkencarbaldimine und Nitroarylalkene, die jeweils vorzugsweise 5 bis 12 C-Atome enthalten.

Das Einsatzmaterial kann weiterhin gegebenenfalls Keto-, Carboxyl-, Carbonsäureester- und/oder Carbonsäureamidgruppen enthalten.

Die mit dem erfindungsgemäßen Verfahren erreichbaren Ausbeuten und Selektivitäten sind sehr hoch. Im allgemeinen liegen die Ausbeuten zwischen 90 und 100 %, die Selektivitäten zwischen 95 und 100 %.

Da das erfindungsgemäße Verfahren im allgemeinen unter Wasserstoffdruck durchgeführt wird, kann der Verlauf der Reaktion leicht am Druckabfall beobachtet werden. Das Ende der Reaktion ist am Druckstillstand

erkennbar. Auch bei weiterer Verlängerung der Reaktionszeit bleibt die hohe Selektivität erhalten. Übliche Reaktionszeiten sind z. B. 30 min. bis 10 Stunden; abhängig vom Katalysator, Katalysatorkonzentration und Substrat können aber sowohl kürzere als auch längere Reaktionszeiten auftreten.

**Beispiele**

**Beispiele 1 bis 9**

In einem gerührten Autoklaven aus rostfreiem Stahl von 0,3 l Inhalt wurde eine 15 %-ige Lösung des Eduktes in Butanon und 0,03 Mol-% des Katalysators vorgelegt, verschlossen und unter Wasserstoffdruck auf die angegebene Temperatur geheizt. Durch regelmäßiges Nachdrücken von Wasserstoff wurde der Druck auf dem angegebenen Wert gehalten. Sobald kein Druckabfall mehr erfolgte, wurde abgekühlt und entspannt. Die Reaktionslösung wurde gaschromatographisch untersucht und destillativ aufgearbeitet, wobei das isolierte Produkt spektroskopisch (IR und NMR) auf eventuell vorhandene hydrierte stickstoffhaltige Gruppen untersucht wurde.

Die Einsatzmaterialien, Reaktionsbedingungen und Ergebnisse waren wie folgt, wobei Cp für Cyclopentadienyl, Ph für Phenyl, MeO für Methoxy und Me für Methyl steht und unter Selektivität die Ausbeute an dem angegebenen Produkt, bezogen auf den Umsatz, bedeutet.

**Beispiel 1**

Edukt: Cyclohex-3-ennitril, Produkt: Cyclohexannitril, Katalysator: RuCl(Cp) $(PPh_3)_2$, Reaktionstemperatur: 120° C, Reaktionsdruck: 130 bar, Umsatz: 100 %, Selektivität: 100 %.

**Beispiel 2**

Edukt: Cyclohex-3-ennitril, Produkt: Cyclohexannitril, Katalysator: RuH(Cp) $(PPh_3)_2$, Reaktionstemperatur: 120° C Reaktionsdruck: 140 bar, Umsatz: 100 %, Selektivität: 100 %.

**Beispiel 3**

Edukt: Cyclohex-3-encarbaldoxim, Produkt: Cyclohexancarbaldoxim, Katalysator: RuCl(Cp) $(PPh_3)_2$, Reaktionstemperatur: 120° C, Reaktionsdruck: 140 bar, Umsatz: 90 %, Selektivität: 100 %.

**Beispiel 4**

Edukt: Cyclohex-3-ennitril, Produkt: Cyclohexannitril, Katalysator: RuCl(1,2,4-$Ph_3C_5Me_2$) $(PPh_3)_2$, Reaktionstemperatur: 120° C, Reaktionsdruck: 120 bar, Umsatz: 100 %, Selektivität: 100 %.

**Beispiel 5**

Edukt: Acrylsäurenitril, Produkt: Propionitril, Katalysator: RuH(Cp) $[(4\text{-MeO-}C_6H_4)_3P]_2$, Reaktionstemperatur: 120° C, Reaktionsdruck: 130 bar, Umsatz: 100 %, Selektivität: 100 %.

**Beispiel 6**

Edukt: Cyclohex-3-en-N-phenylcarbaldimin, Produkt: Cyclohexan-N-phenylcarbaldimin, Katalysator: RuCl(CP) $(PPh_3)_2$, Reaktionstemperatur: 120° C, Reaktionsdruck: 130 bar, Umsatz: 90 %, Selektivität: 98 %.

**Beispiel 7**

Edukt: Cyclohex-3-ennitril, Produkt: Cyclohexannitril, Katalysator: RuCl(Cp) $(PPh_3)_2$, Reaktionstemperatur: 170° C, Reaktionsdruck: 150 bar, Umsatz: 100 %, Selektivität: 100 %.

**Beispiel 8**

Edukt: Cyclohex-3-ennitril, Produkt: Cyclohexannitril, Katalysator: RuCl(Cp) $[(2\text{-Me-}C_6H_4)_3P]_2$, Reaktionstemperatur: 120° C, Reaktionsdruck: 130 bar, Umsatz: 100 %, Selektivität: 100 %.

**Beispiel 9**

Edukt: Cyclohex-3-ennitril, Produkt: Cyclohexannitril, Katalysator: RuCl(Cp) $[(2,4,6\text{-}Me_3C_6H_2)_3P]_2$, Reaktionstemperatur: 120° C, Reaktionsdruck: 120 bar, Umsatz: 100 %, Selektivität: 100 %.

**Beispiele 10 bis 18**

Alle Arbeiten wurden unter Schutzgasatmosphäre (Argon oder Stickstoff) durchgeführt.

**Beispiel 10**

Herstellung von $RuCl(Me_5C_5)(PPh_3)_2$

In einem Glaskolben wurden 4,0 g Triphenylphosphan in 100 ml siedendem Ethanol gelöst und schnell nacheinander 1,0 g $RuCl_3 \cdot 3H_2O$, in 30 ml Ethanol gelöst, und 5,0 g Pentamethylcyclopentadien, in 20 ml Ethanol gelöst, zugegeben. Es wurde noch 6 h unter Rückfluß gekocht, langsam abkühlen gelassen (0° C) und die ausgefallenen Kristalle abgesaugt. Diese wurden mit Wasser, Ethanol, Ether und Mexan gewaschen und im Vakuum getrocknet.
Ausbeute: 1,2 g, Schmp.: 152 - 154° C
IR ($cm^{-1}$): 3060, 1590, 1570, 1480, 1435, 1190, 1120, 1090, 740, 695, 520.

**Beispiel 11**

Herstellung von RuCl(Cp) $[(4\text{-MeO-}C_6H_4)_3P]_2$

In einem Glaskolben wurden 5,4 g $(4\text{-}MeOC_6H_4)_3P$ siedendem Ethanol gelöst und schnell nacheinander 1,0 g $RuCl_3 \cdot 3H_2O$, in 30 ml Ethanol gelöst, und 8,0 g Cyclopentadien, in 20 ml Ethanol gelöst, zugegeben. Es wurde noch 4 h unter Rückfluß gekocht, langsam abkühlen gelassen (5° C) und die ausgefallenen Kristalle abgesaugt. Diese wurden mit Wasser, Ethanol, Ether und Hexan gewaschen und im Vakuum getrocknet.
Ausbeute: 1.0 g, Schmp.: 190° C
IR ($cm^{-1}$): 3040, 1595, 1570, 1500, 1290, 1260, 1180, 1120, 1025, 830, 800, 540.

**Beispiel 12**

Herstellung von RuCl(Cp) $(Ph_2PC_2H_5)_2$

In einem Glaskolben wurden 3,5 g $Ph_2PC_2H_5$ in 100 ml siedendem Ethanol gelöst und schnell nacheinander 1,0 g $RuCl_3 \cdot 3H_2O$, in 30 ml Ethanol gelöst, und 8,0 g Cyclopentadien, in 20 ml Ethanol gelöst, zugegeben. Es wurde noch 1 h unter Rückfluß gekocht, langsam abkühlen gelassen (0° C) und die ausgefallenen Kristalle abgesaugt. Diese wurden mit Wasser, Ethanol, Ether und Hexan gewaschen und im Vakuum getrocknet.
Ausbeute: 1,5 g Schmp.: 182 - 188° C
IR ($cm^{-1}$): 3050, 1590, 1570, 1480, 1435, 1085, 1000, 935, 910, 850, 840, 695, 530, 520, 500.

**Beispiel 13**

Herstellung von RuCl(Cp) [$Ph_2P(CH_2)_4PPh_2$]

In einem Glaskolben wurden 4,8 g $Ph_2P(CH_2)_4PPh_2$ in 100 ml siedendem Ethanol gelöst und schnell nacheinander 1 g $RuCl_3 \cdot 3H_2O$, in 30 ml Ethanol gelöst, und 8,0 g Cyclopentadien, in 20 ml Ethanol gelöst, zugegeben. Es wurde noch 2 h unter Rückfluß gekocht, langsam abkühlen gelassen (5°C) und die ausgefallenen Kristalle abgesaugt. Diese wurden mit Wasser, Ethanol, Ether und Hexan gewaschen und im Vakuum getrocknet.
Ausbeute: 0 4 g, Schmp.: >220°C
IR ($cm^{-1}$): 3060, 1590, 1570, 1480, 1435, 1120, 1090, 1000, 880, 745, 725, 695, 540, 520.

**Beispiel 14**

Herstellung von RuCl(Cp) [$(2,4,6\text{-}Me_3C_6H_2)_3P$]$_2$

In einem Glaskolben wurden 7,1 g $(2,4,6\text{-}Me_3C_6H_2)_3P$ in 100 ml siedendem Ethanol gelöst und schnell nacheinander 1,0 g $RuCl_3 \cdot 3H_2O$, in 30 ml Ethanol gelöst, und 8,0 g Cyclopentadien, in 20 ml Ethanol gelöst, zugegeben. Es wurde noch 6 h unter Rückfluß gekocht, langsam abkühlen gelassen (0°C) und die ausgefallenen Kristalle abgesaugt. Diese wurden mit Wasser, Ethanol, Ether und Hexan gewaschen und im Vakuum getrocknet.
Ausbeute: 0,7 g, Schmp.: >230°C
IR ($cm^{-1}$): 3080, 1700, 1585, 1520, 1430, 1410, 1325, 1290, 1170, 1140, 1100, 1065, 1020, 865, 810, 780, 705, 540.

**Beispiel 15**

Herstellung von RuCl($Ph_5C_5$) $(PPh_3)_2$

In einem Glaskolben wurden 4,0 g Triphenylphosphan in 100 ml siedendem Ethanol gelöst und schnell nacheinander 1,0 g $RuCl_3 \cdot 3H_2O$, in 30 ml Ethanol gelöst, und 5,0 g Pentaphenylcyclopentadien, in 30 ml Ethanol gelöst, zugegeben. Es wurde noch 4 h unter Rückfluß gekocht, langsam abkühlen gelassen (5°C) und die ausgefallenen Kristalle abgesaugt. Diese wurden mit Wasser, Ethanol, Ether und Hexan gewaschen und im Vakuum getrocknet.
Ausbeute: 2,8 g, Schmp.: 230°C (Zersetzung)
IR ($cm^{-1}$): 3060, 1590, 1570, 1480, 1430, 1190, 1090, 1000, 745, 695, 550, 520.

**Beispiel 16**

Herstellung von RuCl(2,4-Dimethyltriphenylcyclopentadienyl) $(PPh_3)_2$

In einem Glaskolben wurden 4,0 g Triphenylphosphan in 100 ml siedendem Ethanol gelöst und schnell nacheinander 1,0 g $RuCl_3 \cdot 3H_2O$, in 30 ml Ethanol gelöst, und 2,3 g 2,4-Dimethyltriphenylcyclopentadien, in 30 ml Ethanol gelöst, zugegeben. Es wurde noch 4 h unter Rückfluß gekocht, langsam abkühlen gelassen (5°C) und die ausgefallenen Kristalle abgesaugt. Diese wurden mit Wasser, Ethanol, Ether und Hexan gewaschen und im Vakuum getrocknet.
Ausbeute: 2,8 g, Schmp.: 196 - 198°C
IR ($cm^{-1}$): 3060, 1590, 1570, 1480, 1435, 1190, 1090, 1000, 740, 695, 540, 520.

**Beispiel 17**

Herstellung von RuCl(Cp) [$(4\text{-}Me_2NC_6H_4)_3P$]$_2$

In einem Glaskolben wurden 6,0 g $(Me_2NC_6H_4)_3P$ in 100 ml siedendem Ethanol gelöst und schnell nacheinander 1,0 g $RuCl_3 \cdot 3H_2O$, in 30 ml Ethanol gelöst, und 6 g Cyclopentadien, in 20 ml Ethanol gelöst, zugegeben. Es wurde noch 4 h unter Rückfluß gekocht, langsam abkühlen gelassen (5°C) und die ausgefallenen Kristalle abgesaugt. Diese wurden mit Wasser, Ethanol, Ether und Hexan gewaschen und im Vakuum getrocknet.

Ausbeute: 1,1 g, Schmp.: >230°C
IR ($cm^{-1}$): 3040, 2890, 2810, 1600, 1550, 1520, 1450, 1340, 1290, 1230, 1205, 1165, 1120, 945, 820, 650, 630, 540, 505.

**Beispiel 18**

Herstellung von RuH(Cp) [$(4\text{-}MeOC_6H_4)_3P]_2$

0,3 g Natrium wurden in 100 ml Methanol gelöst, 1 g RuCl(Cp) [$(4\text{-}MeOC_6H_4)_3P]_2$ zugegeben und die entstandene Suspension 1 h bei Raumtemperatur gerührt. Es wurde weitere 30 min bei 50°C gerührt, mit Eis gekühlt, abgesaugt und die Kristalle mit Ether und Hexan gewaschen.
Ausbeute: 0,9 g, Schmp.: >230°C
IR ($cm^{-1}$): 3060, 1935, 1580, 1480, 1280, 1240, 1160, 1100, 1010, 810, 780.

**Patentansprüche**

1. Verfahren zur selektiven katalytischen Hydrierung von Kohlenstoff-Kohlenstoff-Doppelbindungen in Gegenwart reduzierbarer, stickstoffhaltiger Gruppen, dadurch gekennzeichnet, daß man die Hydrierung in Gegenwart eines katalytisch wirksamen Rutheniumkomplexes durchführt, der der Formel

$RuXL_1(L_2)_2$

entspricht, in der
X für Chlor, Brom, Jod oder Wasserstoff steht,
$L_1$ für einen aromatischen Liganden der Formel

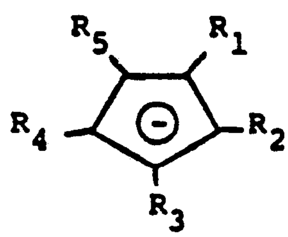

steht, in der $R_1$ bis $R_5$ gleich oder verschieden sein können und für Wasserstoff, Methyl, Ethyl oder Phenyl stehen, wobei auch je zwei benachbarte Reste aus der Gruppe $R_1$ bis $R_5$ gemeinsam einen Kohlenwasserstoffrest derart bilden können, daß $L_1$ insgesamt ein kondensiertes Ringsystem darstellt, das gegebenenfalls unter Reaktionsbedingungen inerte Substituenten enthält
und
$L_2$ für einen Liganden aus der Gruppe der tertiären Organophosphor-, Organoarsen- und Organoantimonverbindungen steht oder
$(L_2)_2$ für einen zweizähnigen Bisphosphan-Liganden steht.

2. Verfahren nach Anspruch 1, dadurch gekennzeichnet, daß $L_1$ für Cyclopentadienyl, Pentamethylcyclopentadienyl, Ethyltetramethylcyclopentadienyl, Pentaphenylcyclopentadienyl, oder Dimethyltriphenylcyclopentadienyl steht.

3. Verfahren nach Ansprüchen 1 und 2, dadurch gekennzeichnet, daß $L_1$ für Cyclopentadienyl steht.

4. Verfahren nach Ansprüchen 1 bis 3, dadurch gekennzeichnet, daß $L_2$ für einen Liganden der Formeln

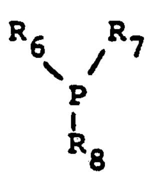 , 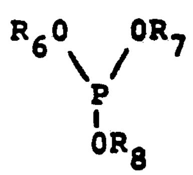 , 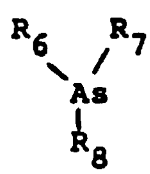 oder 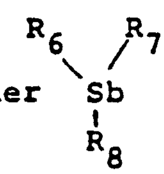

steht, in denen $R_6$, $R_7$ und $R_8$ gleich oder verschieden sein können und Alkyl-, Cycloalkyl-, Aryl- oder Aralkylreste bedeuten, wobei diese Reste gegebenenfalls durch Alkyl-, Hydroxy-, Alkoxy-, Carboalkoxy- oder Halogengruppen substituiert sein können.

5. Verfahren nach Ansprüchen 1 bis 3, dadurch gekennzeichnet, daß $L_2$ für einen Liganden der Formel

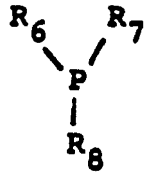

steht.

6. Verfahren nach Ansprüchen 1 bis 5, dadurch gekennzeichnet, daß man pro Mol zu hydrierender C-C-Doppelbindung 0,001 bis 50 mMol eines katalytisch wirksamen Rutheniumkomplexes einsetzt.

7. Verfahren nach Ansprüchen 1 bis 6, dadurch gekennzeichnet, daß man das Verfahren bei Temperaturen im Bereich 20 bis 250° C durchführt.

8. Verfahren nach Ansprüchen 1 bis 7, dadurch gekennzeichnet, daß man das Verfahren bei Drucken im Bereich 5 bis 300 bar durchführt.

9. Verfahren nach Ansprüchen 1 bis 8, dadurch gekennzeichnet, daß man das Verfahren in Gegenwart eines Lösungsmittels durchführt, das man in einem Gewichtsverhältnis von 30 : 1 bis 1 : 10, bezogen aus das Ausgangsprodukt, anwendet.

**Claims**

1. Process for selective catalytic hydrogenation of carbon-carbon double bonds in the presence of reducible, nitrogen-containing groups, characterized in that the hydrogenation is carried out in the presence of a catalytically active ruthenium complex which corresponds to the formula

$RuXL_1(L_2)_2$

in which

X represents chlorine, bromine, iodine or hydrogen,

$L_1$ represents an aromatic ligand of the formula

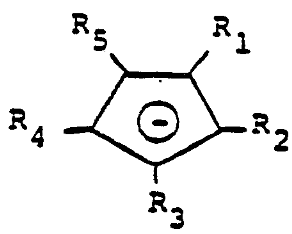

in which

$R_1$ to $R_5$ can be identical or different and represent hydrogen, methyl, ethyl or phenyl, it also being possible for in each case two adjacent radicals from the group $R_1$ to $R_5$ together to form a hydrocarbon radical such that $L_1$ overall represents a fused ring system, which optionally contains substituents which are inert under the reaction conditions, and

$L_2$ represents a ligand from the group comprising tertiary organophosphorus, organoarsenic and organoantimony compounds, or

$(L_2)_2$ represents a bidentate bisphosphane ligand.

2. Process according to Claim 1, characterized in that $L_1$ represents cyclopentadienyl, pentamethylcyclopentadienyl, ethyltetramethylcyclopentadienyl, pentaphenylcyclopentadienyl or dimethyltriphenylcyclopentadienyl.

3. Process according to Claims 1 and 2, characterized in that $L_1$ represents cyclopentadienyl.

4. Process according to Claims 1 to 3, characterized in that $L_2$ represents a ligand of the formulae

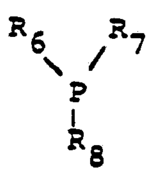 , 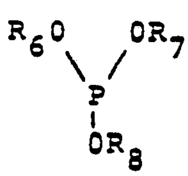 , 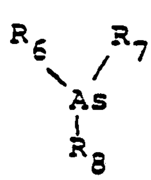 or 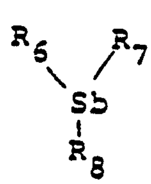

in which

$R_6$, $R_7$ and $R_8$ can be identical or different and denote alkyl, cycloalkyl, aryl or aralkyl radicals, it being

possible for these radicals to be optionally substituted by alkyl, hydroxyl, alkoxy, carbalkoxy or halogen groups.

5. Process according to Claims 1 to 3, characterized in that $L_2$ represents a ligand of the formula

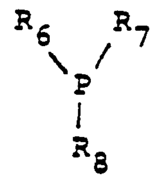

6. Process according to Claims 1 to 5, characterized in that 0.001 to 50 mmol of a catalytically active ruthenium complex are used per mole of C-C double bond to be hydrogenated.

7. Process according to Claims 1 to 6, characterized in that it is carried out at temperatures in the range from 20 to 250° C.

8. Process according to Claims 1 to 7, characterized in that it is carried out under pressures in the range from 5 to 300 bar.

9. Process according to Claims 1 to 8, characterized in that it is carried out in the presence of a solvent which is used in a weight ratio of 30 : 1 to 1 : 10, based on the starting substance.

**Revendications**

1. Procédé d'hydrogénation catalytique sélective de doubles liaisons carbone-carbone en présence de groupes réductibles contenant de l'azote, caractérisé en ce qu'on exécute l'hydrogénation en présence d'un complexe de ruthénium catalytiquement actif qui correspond à la formule:

$RuXL_1 (L_2)_2$

dans laquelle

X représente du chlore, du brome, de l'iode ou de l'hydrogène,

$L_1$ un ligand aromatique de formule:

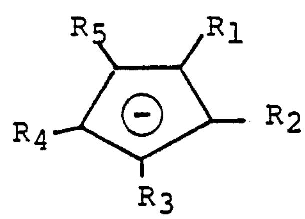

dans laquelle $R_1$ à $R_5$ peuvent être identiques ou différents et représentent de l'hydrogène, méthyle, éthyle ou phényle, deux radicaux voisins du groupe $R_1$ à $R_5$ pouvant aussi former ensemble un radical hydrocarboné en sorte que $L_1$ constitue en tout un système nucléaire condensé qui contient éventuellement des substituants inertes dans les conditions de la réaction et

$L_2$ représente un ligand du groupe des composés tertiaires organophosphore, organoarsenic et organoantimoine ou

$(L_2)_2$ représente un ligand bisphosphane bidentate.

2. Procédé selon la revendication 1, caractérisé en ce que $L_1$ représente un cyclopentadiényle, pentaméthylcyclopentadiényle, éthyltétraméthylcyclopentadiényle, pentaphénylcyclopentadiényle ou diméthyltriphénylcyclopentadiényle.

3. Procédé selon les revendications 1 et 2, caractérisé en ce que $L_1$ représente un cyclopentadiényle.

4. Procédé selon les revendications 1 à 3, caractérisé en ce que $L_2$ représente un ligand de formules:

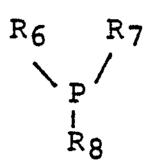 , 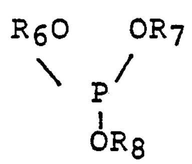 , 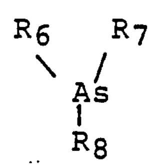 ou 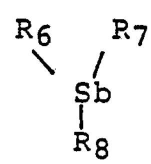

dans lesquelles $R_6$, $R_7$ et $R_8$ peuvent être identiques ou différents et représentent des radicaux alcoyle, cycloalcoyle, aryle ou aralcoyle, ces radicaux pouvant éventuellement être substitués par des groupes alcoyle,

hydroxy, alcoxy, carbalcoxy ou halogène.

5. Procédé selon les revendications 1 à 3, caractérisé en ce que $L_2$ représente un ligand de formule:

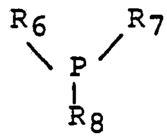

6. Procédé selon les revendications 1 à 5, caractérisé en ce que par mole de double liaison C-C à hydrogéner on utilise 0,001 à 50 mmoles d'un complexe de ruthénium catalytiquement actif.

7. Procédé selon les revendications 1 à 6, caractérisé en ce qu'on exécute le procédé à des températures dans l'intervalle de 20 à 250° C.

8. Procédé selon les revendications 1 à 7, caractérisé en ce qu'on exécute le procédé sous des pressions dans l'intervalle de 5 à 300 bars.

9. Procédé selon les revendications 1 à 8, caractérisé en ce qu'on exécute le procédé en présence d'un solvant que l'on utilise dans un rapport pondéral de 30 : 1 à 1 : 10 par rapport au produit de départ.